# EUROPÄISCHE PATENTSCHRIFT

(11) **EP 1 067 104 B1**
(45) Veröffentlichungstag und Bekanntmachung des Hinweises auf die Patenterteilung: **16.04.2003**
(21) Anmeldenummer: 00113065.7
(22) Anmeldetag: 26.06.2000
(51) Int. Cl.: C07C 7/13

(54) **Trennverfahren**
Separation process
Procédé de séparation

(30) Priorität: 08.07.1999 DE 19931711
(43) Veröffentlichungstag der Anmeldung: 10.01.2001
(73) Patentinhaber: HAARMANN & REIMER GMBH, 37603 Holzminden (DE)
(72) Erfinder: Wöhrle, Ingo, Dr., 37603 Holzminden (DE); Schick, Peter-Reinhard, Dr., 51373 Leverkusen (DE)
(74) Vertreter: Mann, Volker, Dr.

(56) Entgegenhaltungen:
- CHEMICAL ABSTRACTS, vol. 87, no. 5, 1. August 1977 (1977-08-01) Columbus, Ohio, US; abstract no. 39009f, Seite 477; XP002146078 & JP 51 146436 A (KANEGAFUCHI CHEMICAL INDUSTRY CO) 16. Dezember 1976 (1976-12-16)

## Beschreibung

Die Erfindung betrifft ein Verfahren zur Abtrennung von Cycloalkadienen und Cyclopolyenen mit Hilfe von Zeolithen des Faujasit-Typs aus dem bei der Metathese von Cycloalkenen entstandenen Reaktionsgemisch, wobei die Cyclopolyene mindestens zwei Doppelbindungen sowie mindestens 8 Kohlenstoffatome im Ring aufweisen.

Makrocyclische Alkene mit 8 bis 20 Kohlenstoffatomen können als Zwischenprodukte zur Herstellung von Riechstoffen eingesetzt werden. 14 bis 17-gliedrige Cycloalkadiene finden insbesondere Verwendung als Ausgangsprodukte für die Herstellung von Moschus-Riechstoffen. Cyclohexadecenon kann aus Cyclohexadecadien-1,9 hergestellt werden (S. Warwel, H. Bachem, N. Döring, H. Kätker, E. Rose in Seife-Öle-Fette-Wachse 115, 538 (1989)). Generell werden die Ausgangsverbindungen praktisch frei von Verunreinigungen benötigt.

Cycloalkadiene werden üblicherweise durch Metathese von entsprechenden Cyclomonoenen erhalten. Beispielhaft seien Metathesen genannt wie beschrieben in U.S. Pat. No. 3,935,270, Brit. Pat. No. 1,105,565, Brit. Pat. No. 1,118,517, EP 182 333 oder auch Warwel, H. Ridder, G. Hachen in der Chemiker-Ztg. 107, 115 (1983).

Nachteilig an diesen Verfahren ist die große Lösungsmittelmenge, die eingesetzt werden muß, um eine hohe Selektivität an gewünschten Cycloalkadienen zu gewährleisten. Die bekannten Isolierungsverfahren sind mit hohen Energiekosten behaftet und damit teuer. Üblicherweise erfolgt die Isolierung mittels Destillation.

Die Metathese von Cycloocten bzw. Cyclopolyoctenylenen zu Cyclohexadecadien wird durchgeführt wie beispielsweise in EP 182 333 und EP 343 437 beschrieben. In Flüssigphase wird das Eduktgemisch in Gegenwart eines Zinntetraalkyls mit dem heterogenen Katalysatorsystem Re₂O₇ / gamma-Al₂O₃ in Kontakt gebracht.

Die Reaktion wird bevorzugt bei Temperaturen von 20 bis 60°C und mit 0,01 bis 0,05 molaren Lösungen durchgeführt. Die Kontaktzeit am Katalysator beträgt typischerweise 25 bis 200 Sekunden. Die molare Konzentrationsangabe der Lösungen bezieht sich auf die errechneten Cyclomonoeneinheiten, die sich ergeben, wenn man die Cyclopolyene in Monomere aufteilt.

Nach dem eben beschriebenen Verfahren entsteht aus Cycloocten bzw. einem Cycloocten/Cylopolyoctenylen-Gemisch ein Reaktionsgemisch, das im wesentlichen Cyclohexadecadien, Cyclotetracosatrien, Cyclodotriacontatetraen, Cyclotetracontapentaen und Cyclooctatetracontahexaen enthält. Der Anteil an Cyclohexadecadien liegt im allgemeinen im Bereich von 20 bis 50 %, bevorzugt von 25 bis 35 %.

Nach demselben Verfahren entsteht aus Cycloocten und Cyclohepten ein Reaktionsgemisch, das Cyclotetradecadien-1,8, Cyclopentadien-1,8 und Cyclohexadecadien-1,9 sowie höhere makrocyclische Cyclopolyene enthält. Der Anteil an Dienen liegt im allgemeinen im Bereich von 20 bis 50 %, bevorzugt von 25 bis 40 %.

Das Reaktionsgemisch liegt in der Regel in einer Lösung in Metathese-inerten Lösungsmitteln vor.

Lösungsmittel können hier bevorzugt unverzweigte oder cyclische Kohlenwasserstoffe sein.

Als unverzweigte Kohlenwasserstoffe seien hier Verbindungen mit 5 bis 12 Kohlenstoffatomen, bevorzugt 5 bis 8 Kohlenstoffatomen, beispielsweise n-Pentan, und n-Hexan genannt.

Als cyclische Kohlenwasserstoffe seien hier Verbindungen mit 5 bis 8 Kohlenstoffatomen, bevorzugt 5 oder 6 Kohlenstoffatomen, beispielsweise Cyclohexan genannt.

Es ist bekannt, daß kristalline Aluminiumsilikate (Zeolithe) zur Trennung von Kohlenwasserstoffen verwendet werden können. So werden beispielsweise in U.S.-A-3 636 180 großporige Zeolithe zur Trennung Xylolen beschrieben. In U.S.-A-4 313 014 werden diese zur Abtrennung von Cyclohexen eingesetzt.

Es wurde ein Verfahren zur Abtrennung von Cycloalkenen mit mindestens zwei Doppelbindungen sowie mindestens 8 Kohlenstoffatomen im Ring aus einem bei der Metathese von Cycloalkamonoenen bzw. Cyclomonoen/Cyclopolyen-Gemischen entstandenen Reaktionsgemisch in Kohlenwasserstofflösung gefunden. Das Verfahren ist dadurch gekennzeichnet, daß das Reaktionsgemisch mit Zeolithen des Faujasit-Typs in flüssiger Phase behandelt wird, die in der Lösung vorhandenen Olefine mit mindestens zwei Doppelbindungen sowie mindestens 8 Kohlenstoffatomen im Ring vom Zeolithen adsorbiert werden, der beladene Zeolith abgetrennt wird und Cycloalkadiene sowie Cyclopolyene durch Desorption erhalten werden.

Überraschenderweise wurde gefunden, daß Adsorbentien des Typs X und Y in der Alkali- und Erdalkali-Form, sehr geeignete Adsorbentien sind für makrocyclische Verbindungen; die einen kritischen Durchmesser aufweisen, der weitaus größer ist als die Porenöffnung dieser Zeolithe.

Die Cycloalkadiene bzw. Cyclopolyene enthalten bevorzugt Ringgrößen mit mehr als 11 Kohlenstoffatomen.

Desweiteren wurde gefunden, daß genannte Zeolithe eine selektive Adsorption der Cycloalkadiene bewirken. Aus den oben beschriebenen Produktgemischen der Metathese von Cycloocten bzw. Cyclopolyoctenylenen kann eine stufenweise Abreicherung der Diene aus der Rohlösung erreicht werden. Hierbei erfolgt die Abreicherung in der Lösung in der Reihenfolge cis,cis-Cyclohexadecadien-1,9, gefolgt von cis,trans-Cyclohexadecadien- 1,9. Das trans,trans-Cyclohexadecadien-1,9 verbleibt am längsten in der Lösung und reichert sich dort an.

Die Abtrennung von cis,cis-Cyclohexadecadien-1,9 aus hochverdünnten Lösungen gelingt mit Aluminiumsilikaten des X- und Y-Typs in der Natrium oder Ammonium Form. Besonders schonend und effizient gelingt die Adsorption unter Einwirkung von Ultraschallwellen.

Amorphe Adsorbentien wie beispielsweise Kieselgele unterschiedlicher Körnung und Porosität, basische, saure bzw. neutrale Aluminiumoxide oder auch nicht kristalline Aluminiumsilikate diverser Zusammensetzungen bewirkten nicht die gewünschte Adsorption.

Typ X Aluminiumsilikate sind Zeolithe, die eine Faujasit - Kristallstruktur aufweisen. Diese wohlbekannten Zeolithe können mit folgender Formel beschrieben werden:

M_{2/n}O Al₂O₃ xSiO₂ yH₂O

- n:: Wertigkeit des Metalls M
- x:: eine Zahl zwischen 2 und 3
- y:: Kristallwasser

Es kann jeglicher Typ X Aluminiumsilikat verwendet werden, diese Materialien sind kommerziell verfügbar.

Typ Y Aluminiumsilikate sind Zeolithe, die eine Faujasit - Kristallstruktur aufweisen. Diese wohlbekannten Zeolithe können mit folgender Formel beschrieben werden:

M_{2/n}O Al₂O₃ xSiO₂ yH₂O

- n:: Wertigkeit des Metalls M
- x:: eine Zahl zwischen 3 und 6
- y:: Kristallwasser

Es kann jeglicher Typ Y Aluminiumsilikat verwendet werden, diese Materialien sind kommerziell verfügbar.

Vor der Durchführung der Adsorption muß das Kristallwasser aus den Zeolithen entfernt werden. Bei Temperaturen zwischen 150 und 650°C, wahlweise im Vakuum, wird der größte Teil des Wassers aus dem Zeolithen entfernt. Wenn diese Aktivierung bei niedrigeren Temperaturen durchgeführt wird, findet nur partielle Entwässerung statt und die Beladungskapazität des Adsorbens verringert sich.

Zeolithe für das erfindungsgemäße Verfahren sind großporige Zeolithe des Typs X oder Y, die jede Art von Metallkation enthalten können. Die Kationen können auch teilweise gegen Protonen oder Ammoniumionen ausgetauscht sein. Die reine H-Form des Y-Zeolith weist eine hohe katalytische Aktivität auf, die bei höheren Temperaturen zu Isomerisierungen von Doppelbindungen und Kohlenstoffgerüst führen kann. Ebenfalls können zwei oder mehr verschiedene Metallionen ausgetauscht sein. Bevorzugt sind die Kationen der I. oder II. Hauptgruppe des Periodensystems. Besonders bevorzugt sind die Natrium und die Calcium Form.

Die Zeolithe können in Form von Pulvern, Kugeln, Stäbchen, Zylindern, Stangenpreßlingen oder in anderen Formkörpern vorliegen.

Die Adsorption der Cycloalkadiene und Cyclopolyene aus dem Reaktionsgemisch auf den Zeolithen findet bei Temperaturen im Bereich von 0 bis 150 °C, bevorzugt im Bereich von 40 bis 100 °C statt.

Zur Adsorption nach dem erfindungsgemäßen Verfahren werden in der Regel 15 Minuten bis 8 Stunden, bevorzugt 1 bis 4 Stunden benötigt.

Erfindungsgemäß werden in der Regel 5 bis 50 Gew.Teile, bevorzugt 10 bis 25 Gew.Teile Zeolith bezogen auf 1 Gew.Teil Cycloalkene eingesetzt.

Die Desorption erfolgt im allgemeinen mit einer unter Desorptionsbedingungen chemisch und thermisch stabilen Verbindung. Diese wohlfeile Chemikalie muß in einfacher Weise in ausreichender Reinheit vom Extrakt abtrennbar sowie wiedergewinnbar sein.

Erfindungsgemäß wird die Desorption mit aromatischen Kohlenwasserstoffen durchgeführt. Andere Verbindungen wie Ethylacetat oder Isopropanol können ebenfalls verwendet werden, die Desorption geht in diesen Fällen jedoch langsamer von statten. Polare Desorptionsmittel wie beispielsweise Ammoniakwasser, Diole oder auch Aminoalkohole waren nicht erfolgreich.

Als aromatische Kohlenwasserstoffe seien hier Verbindungen mit 6 bis 12 Kohlenstoffatomen, bevorzugt 6 bis 9 Kohlenstoffatomen, wie beispielsweise Benzol, Toluol, Ethylbenzol, Xylol und Trimethylbenzol genannt.

Bei der Desorption sollte die Temperatur nicht deutlich oberhalb von 150°C liegen, da sonst Isomerisierungsprodukte gefunden werden können. Der Zeolith kann sich dann von weiß nach beige verfärben.

Es traten bei dem erfindungsgemäßen Verfahren keine Verfärbung des Zeolithen und keine Isomerisierungsreaktionen der Olefine auf.

Adsorption und Desorption können bei 0 bis 30 bar durchgeführt werden, bevorzugt sind Drücke im Bereich 1 bis 10 bar.

Zur weitgehend vollständigen Desorption werden je nach Formkörper des Zeolithen nach dem erfindungsgemäßen Verfahren in der Regel 15 Minuten bis 20 Stunden, bevorzugt 2 bis 10 Stunden benötigt.

Nach Regenerierung des Adsorbermaterials ist dieses ohne signifikanten Verlust an Adsorptionsfähigkeit wieder einsetzbar. Zur Regenerierung wird der Hauptanteil des noch am Adsorbens anhaftenden Desorptionsmittel im Vakuum entfernt. Nach einer Zeit ist ein Freibrennen des Zeolithen empfehlenswert, da nach einigen Cyclen, je nach Zusammensetzung der Lösung der Olefine, eine partielle Belegung der Oberfläche durch höhere Oligomere bzw. Polymere stattfinden kann.

Das Desorptionsmittel wird bei Drücken zwischen 0 und 1 bar vom Adsorbens entfernt, bevorzugt bei Drücken unter 100 mbar. Die Temperaturen liegt im Bereich von 30 bis 300°C, bevorzugt im Bereich von 100 bis 220°C.

Das Abbrennen des Adsorbens wurde in der Regel bei Temperaturen von 300 bis 700°C, bevorzugt im Bereich von 400 bis 550 °C im Luftstrom durchgeführt.

Das erfindungsgemäße Verfahren kann beispielsweise wie folgt ausgeführt werden:

Eine hochverdünnte Lösung von Cycloalkenen wird in flüssiger Phase in einem inerten Lösungsmittel mit einem entwässerten, d.h. aktivierten, Zeolithen des X- oder Y-Typs für mehrere Stunden in Kontakt gebracht. Das Aluminiumsilikat adsorbiert hierbei diejenigen Substrate, die mindestens zwei Doppelbindungen sowie mindestens 8 Kohlenstoffatome enthalten. Nach beendeter Adsorption wird der beladene Zeolith abfiltriert, gewaschen und getrocknet. Die anschließende Desorption der adsorbierten Substrate erfolgt beispielsweise durch Verdrängung mit aromatischen Kohlenwasserstoffen. Die olefinischen Substrate werden vom Desorptionsmittel befreit und die gewünschten Olefine isoliert.

### Beispiel 1:

Als Adsorbens wurde kommerziell erhältlicher Zeolith NaX (Siliporite 10A, ein Produkt der Firma Elf Atochem / CECA) in Pulverform 12 Stunden bei 450°C an der Luft calciniert.

Es wurden 12 g des aktivierten Zeolithen NaX und eine Lösung von 1 g all-trans 1,5,9-Cyclododecatrien (>99% Reinheit, bezogen von der Firma Aldrich) in 200 ml n-Hexan bei 65°C gerührt. Nach 1 bis 2 Stunden wurde die Lösung auf Raumtemperatur abgekühlt und der Zeolith abgetrennt. Die verbleibende Lösung enthielt <1% der ursprünglichen Menge an Trien.

Für die Desorption wurde der Zeolith in eine Extraktionshülse umgefüllt und in einer Soxhlet-Apparatur 3 Stunden mit 50 ml siedendem Toluol behandelt.

Nach beendeter Desorption wurde der Extrakt vom Toluol destillativ befreit. Es wurden 0,96 g an flüssigem Rückstand erhalten, der all-trans 1,5,9-Cyclododecatrien mit einer GC-Reinheit von >98% enthielt.

### Beispiel 2:

Als Adsorbens wurde kommerziell erhältlicher Zeolith NaX (Siliporite 10A, ein Produkt der Firma Elf Atochem / CECA) in Pulverform 12 Stunden bei 450°C an der Luft calciniert.

Die Adsorption der durch oben beschriebene Metathese einer 0,024 molaren Lösung in n-Hexan eines Cycloocten/Cyclohepten-Gemisches im molaren Verhältnis 1:1 entstandenen Metathese-Rohlösung wurde untersucht (2.49 g Olefine / l).

50 g des aktivierten Zeolith NaX wurden mit 1200 ml einer Metathese-Rohlösung 2 Stunden bei 65°C gerührt.

Nach dieser Zeit waren die Verbindungen mit zwei oder mehr Doppelbindungen fast vollständig (>98%, GC-Kontrolle) adsorbiert.

Der beladene Zeolith wurde abgetrennt und mit etwas n-Hexan nachgewaschen. Das noch anhaftende n-Hexan wurde danach weitgehend bei 50°C / 1 mbar entfernt.

Für die Desorption wurde der Zeolith in eine Extraktionshülse umgefüllt und in einer Soxhlet-Apparatur 5 Stunden mit 100 ml siedendem Toluol behandelt.

Nach beendeter Desorption wurde der Extrakt vom Toluol destillativ befreit. Es wurden 2,72 g an flüssigem Rückstand erhalten, der eine dem Ausgangsgemisch weitgehend identische Produktverteilung aufwies.

### Beispiel 3:

Als Adsorbens wurde kommerziell erhältlicher Zeolith CaX (ein Produkt der Firma Grace Davison) in Pulverform 12 Stunden bei 450°C an der Luft calciniert.

Die Adsorption der durch oben beschriebene Metathese von Cycloocten entstandenen Reaktionslösung (2,4 g/l in n-Hexan) wurde untersucht. Die Reaktionslösung enthielt typischerweise 5% Cyclocten, 30% Cyclohexadecadien-1,9, 33% Triene, 18% Tetraene, 9% Pentaene und 4% Hexaene.

60 g des aktivierten Zeolith CaX wurden mit 1400 ml einer Metathese-Rohlösung 1 bis 3 Stunden bei 65°C gerührt.

Nach dieser Zeit waren die Verbindungen mit zwei oder mehr Doppelbindungen fast vollständig (>98%, GC-Kontrolle) adsorbiert.

Die weitere Isolierung erfolgte wie in Beispiel 2 beschrieben.

Es wurden 3,05 g an flüssigem Rückstand erhalten, der eine dem Ausgangsgemisch weitgehend identische Produktverteilung aufwies.

### Beispiel 4:

Als Adsorbens wurde kommerziell erhältlicher Zeolith NaX (Siliporite 10A, ein Produkt der Firma Elf Atochem / CECA) in Kugelform 12 Stunden bei 450°C an der Luft calciniert.

Die Adsorption der durch oben beschriebene Metathese von Cycloocten entstandenen Reaktionslösung (2,8 g/l in n-Hexan) wurde untersucht. Die Reaktionslösung zeigte die in Beispiel 3 aufgeführte Produktverteilung.

In einen vertikal aufgebauten beheizbaren Rohrreaktor wurden 100 g des aktivierten Zeolithen in Kugelform eingefüllt. Das Zeolithbett wird auf 65°C temperiert. 2000 ml der Metathese-Rohlösung wurden mit Hilfe einer Schlauchpumpe im Kreis gepumpt, wobei das Zeolithbett von unten nach oben vom Adsorbat durchströmt wurde. Die Fördermenge lag bei 2000 bis 5500 ml/h.

Nach 3 bis 5 Stunden waren die Verbindungen mit zwei oder mehr Doppelbindungen fast vollständig (>95%, GC-Kontrolle) adsorbiert.

Nach beendeter Adsorption wurde die Flüssigkeit aus dem Reaktorrohr abgelassen und der beladene Zeolith mit etwas n-Hexan nachgewaschen. Das noch anhaftende n-Hexan wurde danach weitgehend bei 50°C / 1 mbar entfernt.

Zur Desorption wurde das Rohr auf 120°C temperiert. 400 ml Ethylbenzol wurden mit Hilfe einer Schlauchpumpe 5 bis 8 Stunden im Kreis gepumpt, wobei das Zeolithbett von unten nach oben vom Desorptionsmittel durchströmt wurde. Die Fördermenge lag bei 500 bis 2000 ml/h.

Nach beendeter Desorption wird der Extrakt vom Ethylbenzol destillativ befreit. Es wurden 5,06 g an flüssigem Rückstand erhalten, der eine dem Ausgangsgemisch weitgehend identische Produktverteilung aufwies.

### Beispiel 5:

Als Adsorbens wurde kommerziell erhältlicher Zeolith NaY (bezogen von der Firma Strem Chemicals) in Pulverform 12 Stunden bei 450°C an der Luft calciniert.

Die selektive Abreicherung von cis,cis-Cyclohexadecadien-1,9 aus der durch oben beschriebene Metathese von Cycloocten entstandenen Reaktionslösung (2,4 g/l in n-Hexan) wurde untersucht. Die Reaktionslösung enthielt typischerweise 39% Cyclohexadecadien-1,9, 32% Triene, 17% Tetraene, 8% Pentaene und 3% Hexaene.

Die repräsentative Verteilung der Dien-Doppelbindungsisomere lag bei cis,cis : trans, cis : trans,trans = 25 : 58 : 16.

8 g des aktivierten Zeolith NaY wurden mit 500 ml einer Metathese-Rohlösung 30 bis 60 Minuten bei 65°C gerührt.

Nach dieser Zeit waren laut GC >92% des cis,cis-Cyclohexadecadien-1,9 adsorbiert worden.

Nach Abtrennung des beladenen NaY-Materials wurde die verbleibende Lösung einer weiteren Adsorption unterworfen.

Als Adsorbens für die zweite Adsorption wurde kommerziell erhältlicher Zeolith NaX (Siliporite 10A, ein Produkt der Firma Elf Atochem / CECA) in Pulverform 12 Stunden bei 450°C an der Luft calciniert.

20 g des aktivierten Zeolith NaX wurden mit der Metathese-Restlösung 2 bis 3 Stunden bei 65°C gerührt.

Nach dieser Zeit, waren die Verbindungen mit zwei oder mehr Doppelbindungen fast vollständig (>98%, GC-Kontrolle) adsorbiert.

Der beladene Zeolith NaX wurde abgetrennt und mit etwas n-Hexan nachgewaschen. Das noch anhaftende n-Hexan wurde danach weitgehend bei 50°C / 1 mbar entfernt.

Für die Desorption wurde der Zeolith in eine Extraktionshülse umgefüllt und in einer Soxhlet-Apparatur 4 Stunden mit 100 ml siedendem Toluol behandelt.

Nach beendeter Desorption wurde der Extrakt vom Toluol destillativ befreit. Es wurden 1,0 g an flüssigem Rückstand erhalten. Der Gehalt an cis,cis-Cyclohexadecadien-1,9 im Rückstand lag unter 0,5 %.

### Beispiel 6:

Als Adsorbens wurde kommerziell erhältlicher Zeolith NaX (Siliporite 10A, ein Produkt der Firma Elf Atochem / CECA) in Pulverform 12 Stunden bei 450°C an der Luft calciniert.

Die Adsorption der durch oben beschriebene Metathese von Cycloocten entstandenen Reaktionslösung (2,4 g/l in n-Hexan) wurde untersucht. Die Reaktionslösung zeigte die in Beispiel 5 aufgeführte Produktverteilung.

25 g des aktivierten Zeolith NaX wurden mit 500 ml der Metathese-Rohlösung 2 bis 3 Stunden bei 25 bis 30°C im Ultraschallbad in Kontakt gebracht. Danach wurde der Zeolith abfiltriert und das Filtrat gaschromatographisch analysiert.

Die verbleibende Lösung enthielt < 0,5% an cis,cis-Cyclohexadecadien-1,9, es waren >94% dieser Substanz adsorbiert worden.

## Patentansprüche

1. Verfahren zur Abtrennung von Cycloalkenen mit mindestens zwei Doppelbindungen sowie mindestens 8 Kohlenstoffatomen im Ring aus einem bei der Metathese von Cycloalkamonoenen bzw. Cyclomonoen/Cyclopolyen-Gemischen entstandenen Reaktionsgemisch in Kohlenwasserstofflösung, **dadurch gekennzeichnet, dass** das Reaktionsgemisch mit Zeolithen des Faujasit-Typs in flüssiger Phase behandelt wird, die in der Lösung vorhandenen Olefine mit mindestens zwei Doppelbindungen sowie mindestens 8 Kohlenstoffatomen im Ring vom Zeolithen adsorbiert werden, der beladene Zeolith abgetrennt wird und Cycloalkadiene sowie Cyclopolyene durch Desorption erhalten werden.

2. Verfahren nach Anspruch 1, **dadurch gekennzeichnet, dass** Zeolithe des Typs X oder Y eingesetzt werden.

3. Verfahren nach den Ansprüchen 1 und 2, **dadurch gekennzeichnet, dass** eine selektive Adsorption der Cycloalkadiene durch stufenweise Abreicherung der Diene in der Rohlösung erreicht wird.

4. Verfahren nach den Ansprüchen 1 bis 3, **dadurch gekennzeichnet, dass** die Adsorption im Temperaturbereich von 0° bis 150°C durchgeführt wird.

5. Verfahren nach den Ansprüchen 1 bis 4, **dadurch gekennzeichnet, dass** die Adsorption innerhalb von 15 Minuten bis 8 Stunden durchgeführt wird.

6. Verfahren nach den Ansprüchen 1 bis 5, **dadurch gekennzeichnet, dass** 5 bis 50 Gew.-Teile Zeolith, bezogen auf 1 Gew.-Teil Cycloalkene, eingesetzt wird.

## Claims

1. Process for the separation of cycloalkenes having at least two double bonds and at least 8 carbon atoms in the ring from a reaction mixture obtained during the metathesis of cycloalkamonoenes or cyclo-monoene/cyclopolyene mixtures, in hydrocarbon solution, **characterised in that** the reaction mixture is treated with zeolites of the Faujasit type in a liquid phase, the olefins present in the solution and having at least two double bonds and at least 8 carbon atoms in the ring are adsorbed by the zeolite, the loaded zeolite is separated and cycloalkadienes and cyclopolyenes are obtained by desorption.

2. Process according to claim 1, **characterised in that** zeolites of the X or Y type are used.

3. Process according to claims 1 and 2, **characterised in that** selective adsorption of the cycloalkadienes is achieved by a step-wise drop in concentration of the dienes in the raw solution.

4. Process according to claims 1 to 3, **characterised in that** the adsorption is carried out in the temperature range of 0 to 150 °C.

5. Process according to claims 1 to 4, **characterised in that** the adsorption is carried out within 15 minutes to 8 hours.

6. Process according to claims 1 to 5, **characterised in that** 5 to 50 parts by weight of zeolite, based on 1 part by weight of cycloalkenes, are used.

## Revendications

1. Procédé pour séparer des cycloalcènes ayant au moins deux doubles liaisons ainsi qu'au moins 8 atomes de carbone dans le cycle à partir d'un mélange réactionnel formé lors de la métathèse de cycloalcamonoènes ou de mélanges cyclomonoènes/cyclopolyènes en solution dans un hydrocarbure, **caractérisé en ce que** le mélange réactionnel est traité avec des zéolites du type faujasite en phase liquide, les oléfines ayant au moins deux doubles liaisons ainsi qu'au moins 8 atomes de carbone dans le cycle qui sont présentes dans la solution sont adsorbées par la zéolite, la zéolite chargée est séparée et les cycloalcadiènes ainsi que les cyclopolyènes sont obtenus par désorption.

2. Procédé selon la revendication 1, **caractérisé en ce que** l'on utilise des zéolites de type X ou Y.

3. Procédé selon les revendications 1 et 2, **caractérisé en ce que** l'on obtient une adsorption sélective des cycloalcadiènes par appauvrissement par étapes des diènes dans la solution brute.

4. Procédé selon les revendications 1 à 3, **caractérisé en ce que** l'adsorption est réalisée dans le domaine de températures de 0 à 150°C.

5. Procédé selon les revendications 1 à 4, **caractérisé en ce que** l'adsorption est réalisée en 15 min à 8 h.

6. Procédé selon les revendications 1 à 5, **caractérisé en ce que** l'on utilise 5 à 50 parties en masse de zéolite par partie en masse de cycloalcènes.
